# EUROPEAN PATENT SPECIFICATION

(11) **EP 4 340 813 B1**
(45) Date of publication and mention of the grant of the patent: **04.09.2024**
(21) Application number: 22755215.5
(22) Date of filing: 27.07.2022
(51) Int. Cl.: A61K 9/00, A61K 9/08, A61K 47/10, A61P 1/00, A61K 31/4439, A61K 45/06

(54) **USE OF THYROID HORMONE T4 IN A HIGH VERSATILITY TREATMENT IN PATIENTS TAKING PROTON PUMP INHIBITORS**
VERWENDUNG VON SCHILDDRÜSENHORMON T4 IN EINER BEHANDLUNG MIT HOHEN VIELSEITIGEN EIGENSCHAFTEN BEI PATIENTEN MIT PROTONENPUMPENHEMMERN
UTILISATION DE L'HORMONE THYROÏDIENNE T4 DANS UN TRAITEMENT DE GRANDE POLYVALENCE CHEZ DES PATIENTS PRENANT DES INHIBITEURS DE LA POMPE À PROTONS

(30) Priority: 28.07.2021 IT 202100020105
(43) Date of publication of application: 27.03.2024
(73) Proprietor: Altergon S.A., 6900 Lugano (CH)
(72) Inventor: SCARSI PERLER, Claudia, 6900 Lugano (CH); BETTAZZI, Elisa, 6900 Lugano (CH)
(74) Representative: M. Zardi & Co S.A.
(86) International application number: PCT/EP2022/071125
(87) International publication number: WO 2023/006841

(56) References cited:
- WO-A1-2020/178074
- VITA ROBERTO ET AL: "Switching Levothyroxine From the Tablet to the Oral Solution Formulation Corrects the Impaired Absorption of Levothyroxine Induced by Proton-Pump Inhibitors", vol. 99, no. 12, 1 December 2014 (2014-12-01), US, pages 4481 - 4486, XP055910221, ISSN: 0021-972X, Retrieved from the Internet <URL:https://watermark.silverchair.com/jcem4481.pdf?token=AQECAHi208BE49Ooan9kkhW_Ercy7Dm3ZL_9Cf3qfKAc485ysgAAAtowggLWBgkqhkiG9w0BBwagggLHMIICwwIBADCCArwGCSqGSIb3DQEHATAeBglghkgBZQMEAS4wEQQMw2DIiMoFv7UWENaCAgEQgIICjaPahEBhuVKJqMNlui98OeRt7FdMCFh6rvHhgr8DkgbJgmYz3xIFiT2wQsp3xMvaLC8xtnTcwaDmM4DuNDqS1s88mRf> DOI: 10.1210/jc.2014-2684
- SENG YUE CORINNE ET AL: "When bioequivalence in healthy volunteers may not translate to bioequivalence in patients: differential effects of increased gastric pH on the pharmacokinetics of levothyroxine capsules and tablets", JOURNAL OF PHARMACY AND PHARMACEUTICAL SCIENCES, vol. 18, no. 5, 1 January 2015 (2015-01-01), CA, pages 844 - 855, XP055910162, ISSN: 1482-1826, DOI: 10.18433/J36P5M

## Description

### FIELD OF THE INVENTION

The present invention pertains the field of treatment of pathological conditions related to an abnormal functioning of the thyroid hormone, in particular in the case of patients who are already being treated with drugs for other pathologies.

### PRIOR ART

The thyroid hormone T4 (levothyroxine or tetraiodothyronine) is secreted by the follicular cells of the thyroid as a response to the pituitary gland hormone TSH, whose production is in turn regulated by the thyrotropin-releasing hormone TRH. The thyroid hormone T4, as well as its analogue T3 (liothyronine or triiodothyronine), is essential for normal body development of children and for the maturation of the various apparatuses, in particular of the skeleton, and regulates metabolic activity in the adult, influencing the function of every organ and tissue. In particular, it increases oxygen consumption at rest, basal metabolism, body temperature and daily caloric needs; it further regulates carbohydrate metabolism, promoting glycogenolysis and gluconeogenesis and increasing the activity of the enzymes involved in glucose oxidation. The thyroid hormone T4 is also involved in lipolysis and in lipogenesis, regulates protein synthesis, exerts a trophic effect on the muscle and influences the cardiovascular system. It is further essential for heart function, increasing myocardial contractibility (positive inotropic effect), heart rate (positive chronotropic effect) and venous return to the heart.

Pharmaceutical compositions of thyroid hormones, in particular T4, are commonly used for the treatment or the prevention of diseases associated with defective functioning of the thyroid hormone. The treatment typically continues throughout the patient's life and the posology (dose and frequency of administration) should be personalized according to the response of the patient.

Thyroid hormones are conveniently orally administered. In particular, oral solutions result in a full absorption of the administered dose, generally within 1.5 hours from administration. For example, patent application WO2018/073209 describes an alcohol-free oral solution of thyroid hormone T4, with high stability, i. e. being protected against the undesired conversion of T4 into T3; said solution is also subject matter of patent application WO2020/178074 being disclosed therein as showing an absorption not affected by the timing of meals. The mechanism by which meal may interfere with absorption of T4 hormone is not fully elucidated and may depend, inter alia, on the specific food at hand: for example, Liel et al. in J.Clin.Endocrinol.Metab., 1996, 81(2), 857-859 and Jonklass et al. in Thyroid 2014, 24(12), 1670-1751 shows that fiber have a sequestrant action of T4 hormone. Patent application WO2013/072304 describes compositions of thyroid hormone T4 in a water-alcohol-glycerol solution, for oral administration; the presence of alcohol, beside facilitating hormone dissolution and ensuring a good general stability, contributes to the absorption of the composition after oral administration. It is in fact known that small ethanol amounts accelerate the bioavailability of drugs enhancing their dissolution, stimulating gastrointestinal bloodstream and inhibiting metabolism of first-pass effect (Drugs, 18, 1979, p.299-311); the same publication also refers that small ethanol amounts increase acid secretion by the stomach, and it is further known (e. g. from Pharmaceutical Research, 9(1), 1992, p 131-1378) that solubility of hormone T4 is maximized under hyperacidity conditions.

The selection of the correct dose is a critical aspect of thyroid hormonal treatments: underdosing results in low response, while an excessive dose can cause toxic hyperthyroidism symptoms such as tachycardia, sweating, weight loss, nervousness, diarrhea, bone resorption due to activation of osteoclasts and cardiac problems. It is therefore important for patients to rely on formulations which are reliable in terms of dose accuracy.

It is known from the literature that the presence of proton pump inhibitors (PPIs), such as omeprazole, lansoprazole and the like, in the stomach may cause an undesired reduction of absorption of orally administered T4 hormone. For example, Centanni et al., in N.Engl.J.Med.2006;354: 1787-1795, show that therapy with PPIs neutralizes the effect of T4 to the point that, to obtain the desired blood levels of thyroid hormone, the daily dose of T4 should be increased by 37%. Similarly, Sachmechi et al., in Endocr.Pract. 2007;13:345-918, show that, in patients who had already reached a stable level of thyroid hormone after T4 administration, said level was altered after beginning a PPI treatment. The interference of PPIs on T4 hormone absorption is worsened by the fact that the pH alteration caused by PPI administration is typically long-lasting: cf. Katz et al., Therapeutic Advances in Gastroenterololy, 2015, 8(6), 322-330, showing that the gastric pH reaches the value 4 at 2-5 hours since the PPI administration and then remains above 4 during an extended time window of 6-7 hours. As a consequence, the time window when T4 hormone can be administered at the normal acidic pH of the stomach (optimal condition for T4 absorption) is very narrow, and the more so when repeated administrations of PPI inhibitor and/or modified-release formulations of the same are needed, as is the case of patients suffering from gastric ulcer or gastric reflux.

The interference of PPIs on the oral absorption of T4 is thus a serious therapeutic problem, since these drugs are used in a continuous way by a large portion of population: in said patients, it is very difficult to obtain an effective response in an oral treatment with T4 hormone; in these cases, increasing the dose is not a reliable solution since the situation of malabsorption may vary over time and the compensating dose of T4 may be insufficient or, on the contrary, cause an overdosing. On the other hand, the use of an administration route which is different from the oral one and ensures a higher stability of blood levels of thyroid hormone, such as the injection route, entails a clear practical difficulty for the patient, especially considering that T4 treatments are administered once a day and generally continue for long periods or even for the whole life.

According to Vita et al., J.Clin.Endocrinol.Metab., 2014, 99(12):4481-4486), T4 malabsorption caused by proton pump inhibitors is not observed when T4 is administered in the form of an ethanol-glycerol solution (Tirosint oral solution, IBSA Italia S.r.l.); the authors disclose the study as the first study made on T4 in solution, highlight the role of ethanol in facilitating T4 absorption, and do not provide possible indications for the development of other T4 solutions whose absorption would not be affected by PPIs; even the findings of non-interference of PPI on T4 absorption in this study are questionable. In fact, in the implemented protocol, the hormone T4 is administered before (30 minutes or less) the PPI administration: considering that the gastric pH requires 2-5 hours after PPI administration to grow above 4 (cf. Katz et al., op. cit.) it is evident that the unchanged T4 absorption noticed by the authors was largely caused by the fact that the depressing effect of PPI on T4 absorption had still not occurred.

In the light of the above-mentioned problems, new oral formulations of thyroid hormone T4 whose absorption is not affected by PPI administration, and relative medical uses based on said formulations, are still being researched.

### SUMMARY

The present inventors have now discovered that oral formulations of hormone T4 in ethanol-free glycerol solution may be advantageously administered in patients under oral treatment with proton pump inhibitors (PPIs), where the PPIs exert no effects on T4 oral absorption. Therefore, the invention relates to ethanol-free glycerol solutions of thyroid hormone T4 for the use in the treatment or prevention of pathological conditions related to an abnormal functioning of the thyroid hormone, said use being made in patients under PPI treatment. The abnormal functioning of the thyroid hormone is normally consequence of disturbances of the thyroid gland, reflecting in an abnormal secretion of the hormone and altered plasmatic levels of the same, compared to the physiological ones. The new use herein suggested is characterized by a high versatility of application, by allowing the free administration of the two drugs according to any desired regimen, without being subject to limiting schedules to avoid possible interferences between the two drugs. The independence of T4 absorption in the presence of PPI was experimentally verified in stringent conditions, i.e. the hormone T4 was administered to volunteers under chronic PPI administration, at time points when the gastric environment was stably and permanently conditioned by PPI administration; it was then possible to evaluate T4 absorption in conditions being apparently most unfavorable with respect to a possible interference by PPI; nevertheless, even in these stringent conditions, T4 absorption was completely identical to that observed in control subjects treated with T4 only, thus fully supporting the versatility of the present medical use.

### DETAILED DESCRIPTION OF THE INVENTION

In the present description and claims, the terms "thyroid hormone T4", "hormone T4" or simply "T4", which are synonyms according to the invention, mean the thyroxine hormone, also known as levothyroxine or tetraiodothyronine, produced by the thyroid gland; any form of this hormone, whether extractive or synthetic, as such or in the form of a salt or derivative thereof, may be used herein; optionally, it may be used in mixture with the other thyroid hormone (T3, also known as liothyronine or triiodothyronine); the above-mentioned terms "T4" and synonyms are herein optionally meant open to mixtures of T4 with the hormone T3.

The term "glycerol solution" used herein indicates a liquid system whose liquid phase is provided by glycerol and wherein the thyroid hormone T4 is homogeneously dissolved under normal exposure conditions of a pharmaceutical formulation, i.e., between about +10 and about +40°C.

The term "glycerol" indifferently indicates herein pure glycerol or, preferably, the commercially available glycerol, the latter being a solution of glycerol at high concentration in water, typically a concentration of 70-90%, for example 80% or 85%, the rest being water.

The present solution is free of ethanol: it was in fact verified that this component is by no means required to obtain the high oral bioavailability of T4 according to the present invention; this is supported by the tests in the attached experimental section, which were performed in the absence of ethanol and of any other T4 solubilizing additive. In accordance with these data, a variant is provided wherein the solution according to the invention is free, besides of ethanol, of also any other alcohol and/or any other agent solubilizing the hormone T4. In another variant, the solution consists in glycerol as previously defined and hormone T4.

The solution of hormone T4 object of the invention may be used in therapy as a stock solution for oral use, for example as a beverage or syrup; preferably, it is subdivided in dosage units suitable for single and precise oral administrations. The stock solution may be also packaged in a bottle or similar container containing, for example, 10 to 150 mL of said solution. The dosage units are typically provided in a suitable container such as a vial or an ampoule and may contain, in a conventional volume of solution, for example 0.5-10 mL, a standard dose of hormone T4 for a patient, which typically ranges between 10 and 300 µg, for example between 50 and 150 µg; however, thanks to the present invention, it is possible to provide dosage units even much higher, for example in the range 200-800 µg, or 300-800 µg, or 400-800 µg, or 500-800 µg, or 600-800 µg, or 700-800 µg. In general, any dosage unit comprising for example 10 to 800 µg of thyroid hormone T4 may be made according to the invention.

The concentration of hormone T4 in the present solutions depends on the dosage/volume that is intended to be administered and may be extensively varied in that sense, including in particular the above defined dosages of hormone T4 in conventional volume suitable for oral administration, for example 0.5-10 mL. In a non-limiting way, useful concentrations of hormone T4 in the present solutions may be in the range between 10 and 300 µg/mL, for example between 100 and 200 µg/mL.

Proton pump inhibitors, herein abbreviated as PPIs, are a widely recognized class of drugs (see for example Gut Liver. 2017 Jan; 11(1):27-37), and are commonly used in the treatment of a variety of disorders of acidic secretion such as gastric hyperacidity, gastroesophageal reflux, peptic ulcer, etc. Any PPI that can be administered orally falls within the treatment according to the present invention; among the currently available PPIs can be mentioned, by way of a non-limiting example: omeprazole, esomeprazole, lansoprazole, dexlansoprazole, rabeprazole, pantoprazole, ilaprazole, derivatives and/or mixtures thereof. The type of PPI formulation is also not critical and can be selected from those conventionally available, especially solid or liquid, for an oral administration.

In an advantageous variant, the PPI is used in the form of a modified-release formulation and/or in a regimen of chronic treatment, thereby obtaining stable and constant PPI concentrations at the action site; even in said apparently detrimental conditions, wherein the stomach is continuously exposed to the depressive action of PPI against T4 absorption, and without availability of time windows within which the stomach is free of PPI, the present invention fully showed its efficacy, obtaining thyroid hormone levels which were completely identical to those obtained in control subjects who received only T4 in the absence of PPI.

The administered amount of PPI may be extensively varied and depends on the treatment needs required by the patient. It may be in the range between, for example, 5 and 100 mg for single dosage unit for one patient. If a chronic PPI treatment is used, it may be defined as a daily and continuous PPI administration for at least 4 days, typically for 4-10 days, or even for one or more months.

In the present invention, administrations of T4 and PPI are usually distanced, i.e., separated and independent, thereby providing the maximal advantage of allowing to completely adjust the respective treatments, without limitations of administration schedule due to a possible interference on T4 absorption. However, in a variant of the invention, T4 and PPI can also be administrated simultaneously.

In the present medical use, the glycerol solutions of thyroid hormone T4, possibly including PPI, are orally administered to a patient under oral treatment with PPI, for the treatment or prevention of a pathological condition (disease or symptom) related to an abnormal functioning of the thyroid hormone; the thyroid hormone with an abnormal functioning refers herein to TSH. The relative pathological conditions of interest are those known to respond to the treatment with hormone T4 and/or for which the T4 treatment is normally advisable: by way of a non-limiting example, among them can be mentioned: hypothyroidism of any etiology, such as primary (thyroid), secondary (hypophyseal) or tertiary (hypothalamic) hypothyroidism, subclinical hypothyroidism (wherein the levels of peripheral thyroid hormone are within the normal reference laboratory range but the serum levels of thyroid-stimulating hormone (TSH) are slightly high); the present use further extends to the various types of euthyroid goiter, thyroid nodules, chronic lymphocytic thyroiditis (Hashimoto's thyroiditis), multinodular goiter, and as a coadjuvant of surgery and radiotherapy in the management of thyrotropin, as well as in the treatment of thyroid carcinoma.

The term "under PPI treatment", used to define the patient to whom the present use is intended, extensively refers to the fact that the patient is exposed to an effect of the PPI upon T4 administration, independently of when the PPI treatment began; the present use is therefore intended for patients that have already received one or more PPI doses when T4 is administered and that at that moment are totally or partially exposed to an effect of PPI; it also includes patients who start PPI treatment together with the first dose of hormone T4 and patients who start PPI treatment after the first dose of hormone T4. In a very useful variant to fully obtain the benefits of the invention, the patient to be treated with T4 is already in a stable situation of PPI treatment, such as in particular resulting from chronic PPI treatment, possibly administered by a modified-release form. For example, as a non-limitative embodiment of the invention, the administration of T4 may be performed within a time window ranging from 2 to 14 hours after administration of the PPI: this is a timing wherein the gastric pH is most affected by the pH-raising effect of the PPI and the malabsorption of T4 hormone was normally expected. In another non-limitative embodiment of the invention, the administration of T4 is performed in a time window of 2 to 24 hours after administration of the PPI as a modified-release, e.g., sustained release formulation: also in these conditions the gastric pH is most affected by the pH-raising effect of the PPI and the malabsorption of T4 hormone was normally expected.

In all the above-mentioned cases, the oral absorption of T4 is substantially equal to the one obtainable with the same oral dose of T4 in the absence of PPI, therefore resulting totally independent of the PPI effects and thereby achieving the objectives of the present invention.

The invention is now described by way of the following non-limiting examples.

### EXPERIMENTAL PART

### Randomized, open-label, 3-ways cross-over, comparative bioavailability study of sodium levothyroxine administered as a single oral dose of 600 µg in healthy subjects with or without concurrent use of proton pump inhibitor.

### Step I - Drug-drug interaction

The objective of this study is to evaluate if the extent of absorption of 150 µg/mL sodium levothyroxine (T4) oral solution administered as 600 µg oral dose in fasting healthy subjects is affected by the co-administration of a representative proton pump inhibitor (PPI) administered simultaneously (Test 1, Treatment A) or after some time (Test 2, Treatment B) with respect to the administration of T4 only (Reference, Treatment C).

36 healthy adults, with age between 18 and 50 years, men and women occurring in similar proportions, were enrolled in the present study.

In each period, the subjects receive one of the following treatments:
**Treatment A** (Test 1): 1 × 40 mg omeprazole, delayed-release capsule (Sandoz Inc. USA) administered once a day in the morning on days 1-6; moreover, in the morning of day 5, under fasting conditions and at the same time with omeprazole, the oral solution of sodium levothyroxine (TIROSINT^{®}-SOL, IBSA Institut Biochimique SA, Switzerland) is administered, 4 units of 150 µg T4 for a total of 600 µg.
**Treatment B** (Test 2): 1 × 40 mg omeprazole, delayed-release capsule (Sandoz Inc. USA) administered once a day in the evening on days 1-6; moreover, in the morning of day 5, under fasting conditions, the oral solution of sodium levothyroxine (TIROSINT^{®}-SOL, IBSA Institut Biochimique SA, Switzerland) is administered, 4 units of 150 µg T4 for a total of 600 µg.
**Treatment C** (Reference): 4 units of 150 µg T4 for a total of 600 µg (oral solution TIROSINT^{®}-SOL, IBSA Institut Biochimique SA, Switzerland) are administered in the morning of day 5 under fasting conditions.

A total of 19 blood samples are collected in each period for the evaluation of levothyroxine concentration: 0.5 hours, 0.25 hours, pre-dose (0) and 0.5, 1, 1.5, 2, 2.5, 3, 3.5, 4, 5, 6, 8, 10, 12, 16, 24 and 48 hours after the T4 dose. The total (bound and free) levothyroxine was analyzed in serum samples using a validated LC-MS/MS method. The pharmacokinetics parameters: AUC₀₋₄₈ (h*ng/mL), Cₘₐₓ (ng/mL) and Tₘₐₓ (h) are evaluated.

The results obtained are shown in the following table:

**Table 1: Pharmacokinetic parameters concerning serum concentration of total levothyroxine (data adjusted for basal endogenous concentration)**

| | **Tirosint SOL^{®}+omeprazole simultaneous administration** | | | | | **Tirosint SOL^{®}+omeprazole separated administration** | | | |
|---|---|---|---|---|---|---|---|---|---|
| | **(Test A)** | | | | | **(Test B)** | | | |
| **Parameter (unit)** | **N** | **Mean** | **SD** | **CV%** | | **N** | **Mean** | **SD** | **CV%** |
| AUC₀₋₄₈ (h*ng/mL) | 30 | 1598.70 | 271.67 | 16.99 | | 28 | 1561.87 | 308.0 5 | 19.72 |
| Cₘₐₓ (ng/mL) | 30 | 57.49 | 10.80 | 18.78 | | 28 | 58.66 | 12.35 | 21.05 |

| | **N** | **Median** | **Min** | **Max** | | **N** | **Median** | **Min** | **Max** |
|---|---|---|---|---|---|---|---|---|---|
| Tₘₐₓ (h) | 30 | 1.495 | 0.986 | 4.002 | | 28 | 1.486 | 0.986 | 4.017 |
| | | | | | | | | | |

| | **Tirosint SOL^{®} alone Reference (Test C)** | | | | | | | | |
|---|---|---|---|---|---|---|---|---|---|
| **Parameter (unit)** | **N** | **Mean** | **SD** | **CV%** | | | | | |
| AUC₀₋₄₈ (h*ng/mL) | 31 | 1612.69 | 313.60 | 19.45 | | | | | |
| Cₘₐₓ (ng/mL) | 31 | 62.26 | 12.13 | 19.49 | | | | | |

| | **N** | **Median** | **Min** | **Max** | | | | | |
|---|---|---|---|---|---|---|---|---|---|
| Tₘₐₓ (h) | 31 | 1.492 | 0.986 | 6.013 | | | | | |

For inferential analyses, ANOVA was performed on AUC₀₋₄₈ and Cₘₐₓ (ln-transformed by means of comparison of treatments (AC and BC, separately). For both levothyroxine adjusted for basal concentration and for non-adjusted data, 90% confidence intervals for the geometric mean ratio (Treatment A/Treatment C and Treatment B/Treatment C) based on the least squares means of ANOVA of ln-transformed AUC₀₋₄₈ are in the range 80.00% - 125.00%.

**Table 2: Total levothyroxine (data adjusted for basal concentration)-Ratios, 90 % geometric confidence intervals, CV values intra- and inter-subjects - Day 5 - Comparison of Treatment A vs. C**

| **Parameter (unit)** | **Treatm. A** | **Treatm. C** | **Ratio (%)¹** | **90% CI Inf.²** | **90% CI Sup.²** | **CV (%) Intra-subj.** | **CV (%) Inter-subj.** |
|---|---|---|---|---|---|---|---|
| | **LSM** | **LSM** | | | | | |
| | | | | | | | |
| AUC₀₋₄₈ (h*ng/mL) | 1601.44 | 1617.89 | 98.98 | 94.13 | 104.08 | 11.20 | 15.51 |
| Cₘₐₓ (ng/mL) | 57.23 | 62.42 | 91.68 | 86.60 | 97.05 | 12.72 | 15.56 |

| | | | | | | | |
|---|---|---|---|---|---|---|---|
| ¹: Calculated using the least squares method according to the formula e(difference) × 100. ²: Geometric confidence interval using In-transformed data. | | | | | | | |

**Table 3: Total levothyroxine (data adjusted for basal concentration) - Ratios, 90 % geometric confidence intervals, CV values intra- and inter-subjects - Day 5 - Comparison of Treatment B vs. C**

| **Parameter (unit)** | **Treatm. B** | **Treatm. C** | **Ratio (%)¹** | **90% CI Inf.²** | **90% CI Sup.²** | **CV (%) Intra-subj.** | **CV (%) Inter-subj.** |
|---|---|---|---|---|---|---|---|
| | **LSM** | **LSM** | | | | | |
| | | | | | | | |
| AUC₀₋₄₈ (h*ng/mL) | 1544.23 | 1560.71 | 98.94 | 94.99 | 103.07 | 8.78 | 19.16 |
| Cₘₐₓ | 57.94 | 61.05 | 94.90 | 89.86 | 100.22 | 11.76 | 18.17 |
| (ng/mL) | | | | | | | |

| | | | | | | | |
|---|---|---|---|---|---|---|---|
| ¹: Calculated using the least squares method according to the formula e(difference) × 100. ²: Geometric confidence interval using In-transformed data. | | | | | | | |

The data shown in Tables 1-3 demonstrate that the absorption of sodium levothyroxine oral solution, herein tested at 600 µg dose, is not affected by the co-administration of proton pump inhibitor and is independent of the mutual administration schedules.

## Claims

1. An ethanol-free glycerol solution of thyroid hormone T4 for the use in the treatment or prevention of a pathological condition related to the abnormal functioning of the thyroid hormone in a patient under treatment with a proton pump inhibitor, both treatments with hormone T4 and with proton pump inhibitor being performed orally.

2. The solution for the use according to claim 1, wherein the solution is in the form of a dosage unit comprising 10 to 800 µg thyroid hormone T4.

3. The solution for the use according to claim 2, wherein the dosage unit comprises 500 to 800 µg thyroid hormone T4.

4. The solution for the use according to claims 2-3, wherein the dosage unit comprises 0.5 to 10 mL of said solution.

5. The solution for the use according to claim 1, in the form of a multidose stock solution.

6. The solution for the use according to claims 1-5, wherein the proton-pump inhibitor is selected from omeprazole, esomeprazole, lansoprazole, dexlansoprazole, rabeprazole, pantoprazole, ilaprazole, derivatives and/or mixtures thereof.

7. The solution for the use according to claims 1-6, wherein the patient is in a regimen of chronic treatment with the proton pump inhibitor.

8. The solution for the use according to claims 1-7, wherein the proton pump inhibitor is administered in the form of a modified-release solution.

9. The solution for the use according to claims 1-8, wherein the patient receives daily between 5 and 100 mg proton pump inhibitor.

## Patentansprüche

1. Ethanol-freie Glycerinlösung von Thyroidhormon T4 zur Verwendung bei der Behandlung oder Prävention einer pathologischen Bedingung, die mit einer abnormalen Funktion des Thyroidhormons in einem Patienten, der mit einem Protonenpumpeninhibitor behandelt wird, in Zusammenhang steht, wobei die Behandlung sowohl mit dem Hormon T4 als auch mit dem Protonenpumpeninhibitor oral erfolgt.

2. Lösung zur Verwendung gemäß Anspruch 1, wobei die Lösung in Form einer Dosiseinheit umfassend 10 bis 800 µg Thyroidhormon T4 ist.

3. Lösung zur Verwendung gemäß Anspruch 2, wobei die Dosiseinheit 500 bis 800 µg Thyroidhormon T4 umfasst.

4. Lösung zur Verwendung gemäß den Ansprüchen 2-3, wobei die Dosiseinheit 0,5 bis 10 ml der Lösung enthält.

5. Lösung zur Verwendung gemäß Anspruch 1, in Form einer Multidosis-Vorratslösung.

6. Lösung zur Verwendung gemäß den Ansprüchen 1-5, wobei der Protonenpumpeninhibitor aus Omeprazol, Esomeprazol, Lansoprazol, Dexlansoprazol, Rabeprazol, Pantoprazol, Ilaprazol, Derivaten und/oder Gemischen davon ausgewählt ist.

7. Lösung zur Verwendung gemäß den Ansprüchen 1-6, wobei sich der Patient einem chronischen Behandlungsschema mit dem Protonenpumpeninhibitor unterzieht.

8. Lösung zur Verwendung gemäß den Ansprüchen 1-7, wobei der Protonenpumpeninhibitor in Form einer Lösung mit modifizierter Freisetzung verabreicht wird.

9. Lösung zur Verwendung gemäß den Ansprüchen 1-8, wobei der Patient täglich zwischen 5 und 100 mg Protonenpumpeninhibitor erhält.

## Revendications

1. Solution d'hormone thyroïdienne T4 dans le glycérol sans éthanol pour son utilisation dans le traitement ou la prévention d'un état pathologique associé au fonctionnement anormal de l'hormone thyroïdienne chez un patient sous traitement d'un inhibiteur de la pompe à protons, les deux traitements avec l'hormone T4 et avec l'inhibiteur de la pompe à protons étant administrés par voie orale.

2. Solution pour l'utilisation selon la revendication 1, dans laquelle la solution se présente sous la forme d'une unité posologique comprenant 10 à 800 µg d'hormone thyroïdienne T4.

3. Solution pour l'utilisation selon la revendication 2, dans laquelle l'unité posologique comprend 500 à 800 µg d'hormone thyroïdienne T4.

4. Solution pour l'utilisation selon les revendications 2-3, dans laquelle l'unité posologique comprend 0,5 à 10 mL de ladite solution.

5. Solution pour l'utilisation selon la revendication 1, sous la forme d'une solution mère multidose.

6. Solution pour l'utilisation selon les revendications 1-5, dans laquelle l'inhibiteur de la pompe à protons est sélectionné parmi l'oméprazole, l'ésoméprazole, le lansoprazole, le dexlansoprazole, le rabéprazole, le pantoprazole, l'ilaprazole, les dérivés et/ou mélanges de ceux-ci.

7. Solution pour l'utilisation selon les revendications 1-6, dans laquelle le patient suit un traitement chronique avec l'inhibiteur de la pompe à protons.

8. Solution pour l'utilisation selon les revendications 1-7, dans laquelle l'inhibiteur de pompe à protons est administré sous la forme d'une solution à libération modifiée.

9. Solution pour l'utilisation selon les revendications 1-8, dans laquelle le patient reçoit quotidiennement entre 5 et 100 mg d'inhibiteur de pompe à protons.
